# EUROPEAN PATENT APPLICATION

(11) **EP 1 266 958 A1**
(43) Date of publication of application: **18.12.2002**
(21) Application number: 01113515.9
(22) Date of filing: 11.06.2001
(51) Int. Cl.: C12M 1/00, G06F 19/00, C23C 14/06, B01J 19/00

(54) **Bio-chip substrate for the embedding of DNA or protein and its fabrication method**

(71) Applicant: Chang, Tao-Kuang, Taipei Hsien (TW); Liu, Jeng-Fuh, Taipei Hsien (TW); Chen, Chih-Shen, Taipei Hsien (TW)
(72) Inventor: Chang, Tao-Kuang, Taipei Hsien (TW); Liu, Jeng-Fuh, Taipei Hsien (TW); Chen, Chih-Shen, Taipei Hsien (TW)
(74) Representative: Casalonga, Axel

(57) **Abstract**

A bio-chip substrate includes a base material selected from nylon or resin, and a layer of high purity nano microchip diamond membrane bonded to the surface of the base material for the embedding of DNA or protein. The invention relates also to the fabrication of the bio-chip substrate.

## Description

### BACKGROUND OF THE INVENTION

The present invention relates to a bio-chip substrate for the embedding of DNA or protein. The invention relates also to the method of fabricating the bio-chip substrate.

The concept of bio-chip was developed in late of twenty centuries. Many research centers incorporated microelectronics, micro-mechanics, life science, and bionics to form a combined product. The broad definition of bio-chip means the product made on glass, silicon or plastic plate for use in biochemical analysis by means of the application of industrial technology including microelectronics and micro-mechanics. A bio-chip is to be used to act on DNA, protein, or cell structure. The main features of bio-chip technology include high reliability and accuracy of analysis, rapid analysis speed, less amount of sample and reagent consumption, totality (parallelism) of experimental data. Using bio-chip is one of the best ways in studying life science. The most popularly accepted DNA sample preparation method is the microarray technology. According to this microarray technology, a mechanical arm is controlled to implant synthesized DNAs in a glass, silicon, or plastic plate at a high density. The technical bottleneck in using microarray technology to make bio-chips is how to let DNA or protein be embedded in the bio-chip substrate. Glass plates or carrier slides are commonly used for making bio-chip substrates. A glass plate or carrier slide type bio-chip substrate is not the best product for the embedding of DNA or protein because DNA or protein cannot easily positively be embedded in a glass plate or carrier slide type bio-chip substrate.

### SUMMARY OF THE INVENTION

In DNA and protein, peptide bond is the main bonding structure, disulfide bond and hydrogen bond are the secondary bonding structure; hydrophobic property is the third grade structure bonding structure. The main elements for these three structures are carbon, hydrogen, nitrogen, and sulfur. Further, because diamond is composed of carbon atoms, it is attractive to lipoprotein or hydrophobic substance. The present invention has been accomplished under the circumstances in view. According to the present invention, the bio-chip substrate comprises a base material selected from nylon or resin, and a layer of high purity nano microchip diamond membrane bonded to the surface of the base material for the embedding of DNA or protein. The high purity nano microchip diamond membrane

### BRIEF DESCRIPTION OF THE DRAWINGS

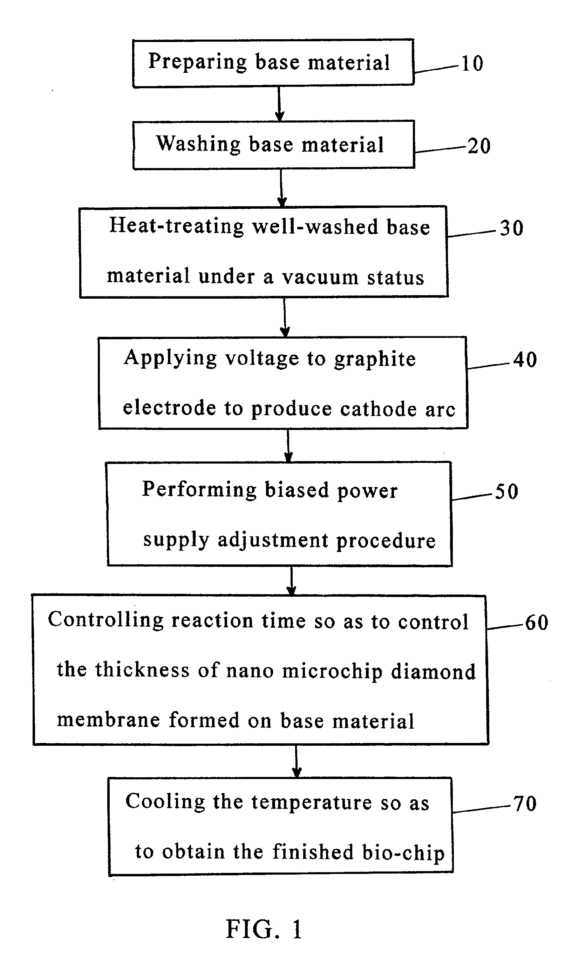

FIG. 1 is a bio-chip substrate fabrication flow chart according to the present invention.

Referring to FIG. 1, the bio-chip substrate fabrication method of the present invention includes the steps of:
10) preparing a base material obtained from nylon or resin for the coating of a medium on the surface thereof;
20) washing the base material to remove dust from the surface of the base material;
30) heat-treating the well-washed base material under a vacuum environment at the reaction temperature of 20∼150°C;
40) preparing a graphite electrode and then applying a voltage to the graphite electrode to produce a cathode arc to further produce free carbon molecules;
50) performing a biased power supply adjustment procedure to trigger charged carbon ions, causing charged carbon ions to be bonded to the surface of the base material to form a layer of high purity nano microchip diamond membrane on the base material;
60) controlling the graphite electrode reaction time under a vacuum environment during the formation of the nano microchip diamond membrane on the base material, so as to control the thickness of the nano microchip diamond membrane within 1∼3 µm;
70) cooling the temperature, so as to obtain a finished bio-chip substrate having a flat surface for the embedding of DNA or protein.

As indicated above, a bio-chip substrate made according to the aforesaid procedure has a layer of high purity nano microchip diamond membrane on the surface thereof. Because of the effect of the layer of high purity nano microchip diamond membrane, the bio-chip substrate has the properties of high corrosion-protective power, high heat conductivity, high light penetrability, and high compatibility to DNA and protein. Because of the aforesaid properties, the micro-chip substrate is practical for use in UV or fluorescent inspection of DNA or protein, and preservation of related sample and chip data.

## Claims

1. A bio-chip substrate fabrication method comprising the steps of:
a. preparing a base material selected from nylon or resin;
b. washing the base material thus prepared to remove dust from the base material;
c. heat-treating the base material thus obtained under a vacuum environment at a predetermined temperature;
d. preparing a graphite electrode and then applying a voltage to the prepared graphite electrode to produce a cathode arc to further produce free carbon molecules;
e. performing a biased power supply adjustment procedure to trigger charged carbon ions, causing charged carbon ions to be bonded to the surface of the base material to form a layer of high purity nano microchip diamond membrane on the base material;
f. controlling the graphite electrode reaction time under a vacuum environment during the formation of the nano microchip diamond membrane on the base material, so as to control the thickness of the nano microchip diamond membrane; and
g. cooling the temperature, so as to obtain a finished bio-chip substrate.

2. The bio-chip substrate fabrication method of claim 1 wherein the step c. of heat-treating the base material thus obtained under a vacuum environment at a predetermined temperature is to heat-treat the base material at the reaction temperature of 20∼150°C.

3. The bio-chip substrate fabrication method of claim 1 wherein the thickness of said nano microchip diamond membrane is controlled within 1∼3 µm.

4. A bio-chip substrate comprising a base material selected one of a set of materials including nylon and resin, and a layer of high purity nano microchip diamond membrane bonded to the surface of said base material for the embedding of DNA or protein.
